# EUROPÄISCHE PATENTSCHRIFT

(11) **EP 3 046 590 B1**
(45) Veröffentlichungstag und Bekanntmachung des Hinweises auf die Patenterteilung: **11.10.2017**
(21) Anmeldenummer: 15734058.9
(22) Anmeldetag: 15.06.2015
(51) Int. Cl.: A61K 49/10

(54) **VERFAHREN ZUM HERSTELLEN EINER FLÜSSIGEN PHARMAZEUTISCHEN ZUBEREITUNG**
METHOD FOR PRODUCING A LIQUID PHARMACEUTICAL PREPARATION
PROCÉDÉ DE FABRICATION D'UNE PRÉPARATION PHARMACEUTIQUE LIQUIDE

(30) Priorität: 31.07.2014 AT 6082014
(43) Veröffentlichungstag der Anmeldung: 27.07.2016
(73) Patentinhaber: Sanochemia Pharmazeutika AG, 1090 Wien (AT)
(72) Erfinder: WELZIG, Stefan, 1030 Wien (AT); SCHUECKER, Raffael, 1020 Wien (AT); KÄLZ, Beate, 7035 Steinbrunn (AT); GUNGL, József, 9423 Ágfalva (HU); GERDES, Klaus, 40233 Düsseldorf (DE); BRAUNRATH, Roswitha, 7221 Marz (AT)
(74) Vertreter: Beer & Partner Patentanwälte KG
(86) Internationale Anmeldenummer: PCT/AT2015/000088
(87) Internationale Veröffentlichungsnummer: WO 2016/015066

(56) Entgegenhaltungen:
- EP-A1- 0 263 861
- EP-B1- 2 242 515
- US-A- 5 560 903
- US-A1- 2012 082 624

## Beschreibung

Die Erfindung betrifft ein Verfahren zum Herstellen einer flüssigen pharmazeutischen Zubereitung, die einen Komplex aus dem makrozyklischen Chelat DOTA (1,4,7,10-Tetraazacyclododecan-1,4,7,10-tetraessigsäure) mit Gadolinium und zusätzlich als Base L-Lysin oder Meglumin enthält.

Die erfindungsgemäß herstellbare Zubereitung kann als Kontrastmittel verwendet werden.

Aus der EP 2 242 515 B1 ist ein Verfahren der gattungsgemäßen Art bekannt, das beispielsweise zur Herstellung von Gadotersäure-Megluminsalz dient.

Das aus der EP 2 242 515 B1 bekannte Verfahren zum Herstellen einer flüssigen pharmazeutischen Formulierung, die einen Komplex von makrocyclischem Chelat mit einem Lanthanid enthält, umfasst folgende Schritte:
- Herstellen einer flüssigen pharmazeutischen Zusammensetzung, die den Komplex von makrocyclischem Chelat mit einem Lanthanid und freies makrocyclisches Chelat, das nicht in der Form eines Hilfsstoffs X[X',L] vorliegt, wobei L das makrocyclische Chelat ist und X und X' ein Metallion sind, und freies Lanthanid enthält, durch Mischen einer Lösung von freiem makrocyclischen Chelat und freiem Lanthanid, um so Komplexierung des Lanthanids durch das makrocyclische Chelat zu erhalten, wobei die Mengen an freiem makrocyclischen Chelat und an freiem Lanthanid so sind, dass nicht das gesamte Lanthanid komplexiert wird;
- Messen der Konzentration an freiem Lanthanid in der so erhaltenen pharmazeutischen Formulierung, wobei die Konzentration des freien makrocyclischen Chelats gleich 0 ist;
- Einstellen der Konzentrationen an freiem Chelat und freiem Lanthanid durch Zugeben der Menge an freiem makrocyclischen Chelat zu der so erhaltenen Formulierung, die notwendig ist, um erstens die Komplexierung des freien Lanthanids abzuschließen, um kein freies Lanthanid zu erhalten, und zweitens, um die Zielkonzentration des freien makrocyclischen Chelats in der fertigen flüssigen pharmazeutischen Formulierung zu erreichen, wobei die Menge an freiem makrocyclischen Chelat in der fertigen, flüssigen, pharmazeutischen Formulierung dem Anteil an freiem makrocyclischen Chelat bezogen auf die Menge an komplexiertem makrocyclischen Chelat in der fertigen flüssigen pharmazeutischen Formulierung entspricht.

Das aus der EP 2 242 515 B1 bekannte Verfahren ist vergleichsweise aufwändig und im gewerblichen Maßstab nur schlecht anwendbar, da zahlreiche Nachjustierungsschritte erforderlich sind.

Ein Nacharbeiten des in Beispiel 2 der EP 2 242 515 B1 beschriebenen Verfahrens liefert darüberhinaus kein in der EP 2 242 515 B1 beschriebenes Produkt, da die Komplexierung bei strenger Befolgung der Verfahrensschritte aus Beispiel 2 nur zu rund 88 % erfolgt. Restliches Gadolinium und DOTA bleibt somit unkomplexiert und die in Anspruch 1 unter 1c genannte Spezifikation auf C_{ch 1} gleich 0 (Konzentration an freiem makrozyklischem Chelat gleich Null) wird nicht erreicht. Siehe hierzu das Vergleichsbeispiel.

Da die komplexbildenden Komponenten in komplexierter und freier Form vorliegen, ist ein Einstellen von überschüssigem Chelat, wie in Punkt 1d von Anspruch 1 der EP 2 242 515 B1, dargelegt nicht möglich.

Der Erfindung liegt die Aufgabe zugrunde, ein Verfahren der eingangs genannten Gattung zur Verfügung zu stellen, das einfacher durchzuführen ist und zur gewünschten pharmazeutischen Zubereitung, beispielsweise Gadotersäure-Megluminsalz führt.

Erfindungsgemäß wird die Aufgabe mit einem Verfahren der eingangs genannten Gattung gelöst, welches die folgenden Schritte umfasst:
a) Herstellen einer wässrigen Lösung enthaltend freies DOTA, freies Gadolinium und als Base L-Lysin oder Meglumin.
b) Bestimmen der Gehalte der nach Schritt a) erhaltenen Lösung an freiem DOTA und an freiem Gadolinium.
c) Zugeben von freiem Gadolinium oder freiem DOTA um in der Lösung einen stoechiometrischen Überschuss an freiem DOTA einzustellen, wobei das Zugeben von freiem DOTA und von freiem Gadolinium derart erfolgt, dass in der Zubereitung kein freies Gadolinium vorliegt,
d) Ausführen der Komplexierung bei erhöhter Temperatur.
e) Zugabe von weiterer Base, um den pH-Wert einzustellen.
f) Einstellen des Endvolumens der Zubereitung.

Es ist erkennbar, dass das erfindungsgemäße Verfahren wegen der besonderen Führung des Verfahrens einfach auszuführen ist und in überraschender Weise zu einer Zubereitung führt, die als Kontrastmittel einsetzbar ist, ohne dass die Gefahr besteht, dass in dem Kontrastmittel freies Gadolinium enthalten ist.

Ein Merkmal des Verfahrens gemäß der Erfindung ist die Reaktion von DOTA mit Gadolinium unter Anwesenheit einer Base. Bei der Erfindung werden vor dem ersten Messen des Gehaltes an freiem DOTA und freiem Gadolinium die drei Komponenten DOTA, Gadolinium und L-Lysin oder Meglumin (im Reaktor) gemeinsam zur Reaktion gebracht. Erst durch die Anwesenheit einer Base wird nämlich ein Milieu erhalten, das die Komplexierungsreaktion vervollständigt und dazu führt, die im Unterschuss enthaltene komplexbildende Komponente (Gadolinium) vollständig zu komplexieren, sodass der Gehalt an freiem Gadolinium gleich Null ist. Durch Messen des Überschusses an freiem DOTA kann Gadolinium derart nachdosiert werden, dass der Gehalt an freiem DOTA auf einen Bereich von 200 - 1500 ppm, bezogen auf den Komplex, eingestellt werden kann.

In einer Ausführungsform des erfindungsgemäßen Verfahrens kann so vorgegangen werden, dass zum Herstellen der Lösung gemäß Schritt a) zunächst DOTA in Wasser bei erhöhter Temperatur gelöst wird und dann Gadolinium zugegeben wird.

In einer Ausführungsform des erfindungsgemäßen Verfahrens kann so vorgegangen werden, dass das Zugeben von freiem DOTA und von freiem Gadolinium derart erfolgt, dass in der Zubereitung kein freies Gadolinium vorliegt.

In einer Ausführungsform des erfindungsgemäßen Verfahrens kann so vorgegangen werden, dass das Komplexieren durch weiteres Zugeben einer Base wie L-Lysin oder Meglumin vervollständigt wird.

In einer Ausführungsform des erfindungsgemäßen Verfahrens kann so vorgegangen werden, dass Schritt a) bei einer Temperatur zwischen 60°C und 95°C ausgeführt wird.

In einer Ausführungsform des erfindungsgemäßen Verfahrens kann so vorgegangen werden, dass die Lösung beim Ausführen der Schritte a) und/oder d) gerührt wird. In einer Ausführungsform des erfindungsgemäßen Verfahrens kann so vorgegangen werden, dass als Base Meglumin oder L-Lysin verwendet wird.

In einer Ausführungsform des erfindungsgemäßen Verfahrens kann so vorgegangen werden, dass in Schritt c) das Zugeben von Gadolinium in zwei oder mehr als zwei Teilmengen erfolgt.

In einer Ausführungsform des erfindungsgemäßen Verfahrens kann so vorgegangen werden, dass freies DOTA in Form einer Lösung und/oder freies Gadolinium in Form einer Lösung zugegeben wird.

In einer Ausführungsform des erfindungsgemäßen Verfahrens kann so vorgegangen werden, dass das Gadolinium als Oxid (Gd₂O₃) zugegeben wird. In diesem Fall liegt Gadolinium als dreiwertiges Kation vor.

In einer Ausführungsform des erfindungsgemäßen Verfahrens kann so vorgegangen werden, dass die Konzentration an freiem DOTA in der Zubereitung 180 - 2000 ppm, vorzugsweise 200 bis 1500 ppm, beträgt.

In einer Ausführungsform des erfindungsgemäßen Verfahrens kann so vorgegangen werden, dass die Konzentration an freiem Gadolinium in der Zubereitung unter 10 ppm liegt.

In einer Ausführungsform des erfindungsgemäßen Verfahrens kann so vorgegangen werden, dass der pH-Wert in Schritt e), insbesondere bei Raumtemperatur, auf einen Wert zwischen 7,0 und 7,2 eingestellt wird.

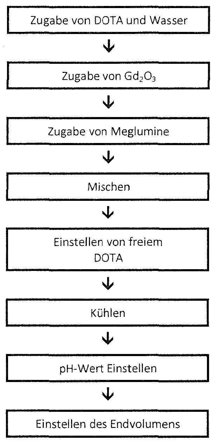

In der Figur ist der grundsätzliche Verfahrensablauf des erfindungsgemäßen Verfahrens wiedergegeben.

Beim Ausführen des erfindungsgemäßen Verfahrens zum Herstellen einer flüssigen, pharmazeutischen Zubereitung durch Komplexieren von DOTA mit Gadolinium können beispielsweise die folgenden Schritte ausgeführt werden:
In Wasser für Injektionszwecke wird DOTA bei einer erhöhten Temperatur gelöst.
In einem weiteren Schritt wird eine vorher berechnete Menge an Gadolinium zugegeben, wobei darauf geachtet wird, dass die berechnete Menge an Gadolinium unterstoechiometrisch sein muss, d.h. dass DOTA in stoechiometrischem Überschuss vorliegt.

Das Komplexieren erfolgt bei hohen Temperaturen über einen Zeitraum der hinreicht, um aus dem Gadolinium und dem DOTA einen Komplex zu bilden.

Die Reaktion der Substanzen (DOTA und Gadolinium) wird durch Zugabe einer dafür erforderlichen Base (beispielsweise Meglumin oder L-Lysin) vervollständigt.

In der Folge wird die Konzentration an freiem DOTA und an freiem Gadolinium bestimmt, um sicherzustellen, dass eine unterstoechiometrische Menge an Gadolinium und eine höhere Konzentration an DOTA vorliegt.

Durch weiteres Einstellen der Konzentrationen der oben genannten freien Anteile (Gadolinium und DOTA) wird die Konzentration so gesteuert, dass die Konzentration an freiem Gadolinium in der fertigen Zubereitung gleich Null ist und die Konzentration an freiem DOTA zwischen 200 und 1500 ppm, berechnet auf den Komplex, vorliegt.

Das Bilden des Komplexes kann bei einer Temperatur im Bereich zwischen 60°C und 95°C ausgeführt werden.

Die Reaktionszeiten für das Bilden des Komplexes hängen vom Verfahrenslauf ab. Beispielsweise wird zwei Stunden lang gerührt und dann zehn Stunden stehen gelassen.

Das Einstellen des Gehaltes an freiem DOTA und des Gehaltes an freiem Gadolinium kann nicht nur mit Hilfe von Festsubstanzen, sondern auch mit Hilfe vorher hergestellter Lösungen der beiden Substanzen vorgenommen werden.

Nachstehend wird das Vergleichsbeispiel, bei dem die Arbeitsweise von Beispiel 2 der EP 2 242 515 B1 angewendet wurde, wiedergegeben.

Der Ansatz aus Beispiel 2 der EP 2 242 515 B1 wurde um den Faktor 500 verkleinert, was einer Laboransatzgröße von 200 ml Gesamtvolumen entspricht.

Das Verhältnis der Einwaagen der Rohstoffe wurde nicht verändert.

Nach Mischen von DOTA und Gadoliniumoxid in Wasser bei 80 °C wurde eine Lösung erhalten. Innerhalb von 48 Stunden wurden mehrere Proben gezogen und der Gehalt an freiem DOTA und an Komplex bestimmt. Es stellte sich heraus, dass einerseits zu keiner Zeit der Gehalt an Komplex größer als 88 % war und andererseits korrelierend hierzu der Gehalt an freiem DOTA unter 11 % lag (siehe Tabelle 1). Aus der Ansatzberechung ergibt sich ein Überschuss an Gadoliniumoxid, weshalb bei vollständiger Komplexierung ein Gehalt von freiem DOTA gleich Null zu erwarten wäre. Da jedoch ein deutlicher Anteil an unkomplexiertem DOTA im Ansatz vorlag, war die Komplexierung nicht vollständig. Eine

Einstellung des freien DOTA war deshalb zu diesem Zeitpunkt, wie in EP 2 242 515 B1 Beispiel 2 Step 3 beschrieben, nicht möglich. Erst durch Zugabe von Meglumin wurde ein Milieu erzeugt, welches die Komplexierung vervollständigte und den Gehalt an Komplex deutlich erhöhte (siehe Tabelle 1).

Viel wichtiger ist jedoch, dass generell erst nach Zugabe von Meglumin eine Gehaltseinstellung an freiem DOTA innerhalb der gewünschten Grenzen möglich ist, da durch die Megluminzugabe das bereits in Lösung vorliegende freie DOTA zur Gänze komplexiert wird (freies DOTA < 50 ppm).

Die Ergebnisse dieses Vergleichsbeispiels widersprechen somit der Lehre von EP 2 242 515 B1.

**Tabelle 1. Messwerte des Vergleichsbeispiels.**

| Reaktionszeit nach Mischen von DOTA und Gadoliniumoxid | Gehalt an freiem DOTA | Gehalt an Komplex |
|---|---|---|
| 1 h | 11.4 % | |
| 2 h | 11.4 % | |
| 3 h | 11.4 % | |
| 4 h | 11.5 % | |
| 8 h | 11.9 % | |
| 24 h | 12.5 % | 234 mg/ml |
| 31 h | 13.6 % | 230 mg/ml |
| 48 h | 15.8 % | 229 mg/ml |
| 48 h; nach Zugabe von Meglumin | < 50 ppm | 262 mg/ml |

Das erfindungsgemäße entwickelte Verfahren löst dieses Problem, indem Meglumin bereits vor der ersten Gehaltsbestimmung von freiem DOTA und Komplex zum Reaktionsansatz zugegeben wird. Es wird ein stoechiometrischer Überschuss an DOTA eingesetzt, wodurch eine vollständige Umsetzung von Gadolinium zum Komplex gewährleistet ist und der Gehalt an freiem Gadolinium bei der ersten Gehaltsbestimmung gleich Null ist. Anhand der Messwerte aus Tabelle 2 ist deutlich erkennbar, dass erst die Zugabe von Meglumin ein Milieu erzeugt, welches die Komplexierung vervollständigt. Eine Gehaltseinstellung von freiem DOTA durch Zugabe von Gadolinium ist dann problemlos möglich (siehe Tabelle 2).

**Tabelle 2. Messwerte des erfindungsgemäßen Verfahrens.**

| Verfahrensschritt | Gehalt an freiem DOTA | Gehalt an Komplex |
|---|---|---|
| Mischen von DOTA und Gadoliniumoxid | 12.7 % | 252 mg/ml |
| 30 min nach Zugabe von Meglumin | 2.1 % | 283 mg/ml |
| 60 min nach Zugabe von Meglumin | 1.7 % | |
| 90 min nach Zugabe von Meglumin | 0.15 % | |
| 60 min nach Zugabe von Gadolinium | 0.11 % | |
| 90 min nach Zugabe von Gadolinium | 0.10 % | 280 mg/ml |
| Einstellen des Endvolumens | 994 ppm | 278 mg/ml |
| Ende der Herstellung, nach 18 h | 981 ppm | 278 mg/ml |

Nachstehend werden Ausführungsbeispiele des erfindungsgemäßen Verfahrens wiedergegeben.

### Beispiel 1:

In 150 ml Wasser mit einer Temperatur von 75 °C wurden 40,5 g DOTA suspendiert. Es wurden 17,8 g Gadoliniumoxid zugegeben und der Ansatz bei 75 °C für 2 Stunden gerührt. Die entstandene Lösung wurde mit 19,5 g Meglumin versetzt und bei 75 °C für eine Stunde gerührt. Danach wurde der Gehalt an freiem DOTA, freiem Gadolinium und Komplex bestimmt und der finale Gehalt an überschüssigem freiem DOTA eingestellt. Die Konzentration an freiem Gadolinium war gleich Null und die Konzentration an überschüssigem freiem DOTA wurde auf einen Wert zwischen 200 und 1500 ppm, berechnet auf den Komplex, eingestellt. Der Reaktionsansatz wurde auf ein Gesamtvolumen von 200 ml aufgefüllt und filtriert.

### Beispiel 2:

In 150 ml Wasser mit einer Temperatur von 75 °C wurden 7,8 g Gadoliniumoxid suspendiert. Es wurden 40,5 g DOTA zugegeben und der Ansatz bei 75 °C für 2 Stunden gerührt. Weitere 10,0 g Gadoliniumoxid wurden zugegeben und erneut bei 75 °C für 15 Minuten gerührt. Die entstandene Lösung wurde mit 19,5 g Meglumin versetzt und bei 75 °C für eine Stunde gerührt. Danach wurde der Gehalt an freiem DOTA, freiem Gadolinium und Komplex bestimmt und der finale Gehalt an überschüssigem freiem DOTA eingestellt. Die Konzentration an freiem Gadolinium war gleich Null und die Konzentration an überschüssigem freiem DOTA wurde auf einen Wert zwischen 200 und 1500 ppm, berechnet auf den Komplex, eingestellt. Der Reaktionsansatz wurde auf ein Gesamtvolumen von 200 ml aufgefüllt und filtriert.

### Beispiel 3:

In 150 ml Wasser mit einer Temperatur von 75 °C wurden 13,5 g DOTA suspendiert. Es wurden 5,9 g Gadoliniumoxid zugegeben und der Ansatz bei 75 °C für 30 Minuten gerührt. Weiters wurden 13,5 g DOTA und 5,9 g Gadoliniumoxid zugegeben und bei 75 °C für 30 Minuten gerührt. Noch einmal wurden 13,5 g DOTA und 6,0 g Gadoliniumoxid zugegeben und bei 75 °C für 30 Minuten gerührt. Die entstandene Lösung wurde mit 19,5 g Meglumin versetzt und bei 75 °C für eine Stunde gerührt. Danach wurde der Gehalt an freiem DOTA, freiem Gadolinium und Komplex bestimmt und der finale Gehalt an überschüssigem freiem DOTA eingestellt. Die Konzentration an freiem Gadolinium war gleich Null sein und die Konzentration an überschüssigem freiem DOTA wurde auf einen Wert zwischen 200 und 1500 ppm, berechnet auf den Komplex, eingestellt. Der Reaktionsansatz wurde auf ein Gesamtvolumen von 200 ml aufgefüllt und filtriert.

Wie in den Beispielen gezeigt, kann beim erfindungsgemäßen Verfahren der finale Gehalt an überschüssigem DOTA auf 200 - 1500 ppm eingestellt werden.

Der Gehalt an freiem Gadolinium ist beim Ausführen des erfindungsgemäßen Verfahrens immer Null, da mit einem Überschuss an DOTA gearbeitet wird. Es wird beim erfindungsgemäßen Verfahren Gadolinium zugegeben, um den Anteil an DOTA auf den genannten Bereich einzustellen.

Beim Ausführen des erfindungsgemäßen Verfahrens können die Substanzen Gadolinium und DOTA abwechselnd zugesetzt werden. Beispielsweise wird je ein Drittel der Sollmenge in halbstündigen Abständen zugegeben.

Am Beginn des erfindungsgemäßen Verfahrens kann Gadolinium vorgelegt werden. Anschließend wird ein Teil, z.B. ein Viertel der Sollmenge an DOTA zugegeben. Die so erhaltene Lösung wird beispielsweise bei 80°C gerührt, z.B. 2 Stunden lang, und dann zu der Lösung die restliche Menge an DOTA zugegeben.

### Zusammenfassend kann ein Ausführungsbeispiel der Erfindung wie folgt beschrieben werden:

Ein Verfahren zum Herstellen einer flüssigen, pharmazeutischen Zubereitung, die einen Komplex aus Gadolinium und DOTA sowie eine Base wie L-Lysin oder Meglumin enthält, umfasst beispielsweise die folgenden Schritte:
a) Es wird eine wässrige Lösung, bestehend aus DOTA, Gadolinium und einer Base wie Meglumin oder L-Lysin hergestellt.
b) Der Gehalt an freiem DOTA und freiem Gadolinium in der nach Schritt a) erhaltenen Lösung wird bestimmt.
c) Es wird freies Gadolinium und/oder freies DOTA zugegeben um den Gehalt an freiem DOTA auf 200 - 1500ppm, bezogen auf den Komplex, einzustellen.
d) Die Komplexierung kann bei erhöhter Temperatur ausgeführt werden.
e) Zum Einstellen des finalen pH-Wertes wird weitere Base zugegeben.
f) Das Endvolumen der Zubereitung wird eingestellt.

## Patentansprüche

1. Verfahren zum Herstellen einer flüssigen, pharmazeutischen Zubereitung, die einen Komplex aus DOTA und Gadolinium sowie eine Base, wie beispielsweise L-Lysin oder Meglumin, enthält, **gekennzeichnet durch** die folgenden Schritte:
a) Es wird eine wässrige Lösung aus DOTA, Gadolinium und einer Base wie Meglumin oder L-Lysin hergestellt,
b) in der gemäß Schritt a) erhaltenen Lösung wird der Gehalt an freiem DOTA und an freiem Gadolinium bestimmt,
c) es wird freies Gadolinium und/oder freies DOTA zugegeben, um in der gemäß Schritt a) erhaltenen Lösung einen Gehalt an freiem DOTA von 200 - 1500 ppm, bezogen auf den Komplex, einzustellen, wobei das Zugeben von freiem DOTA und von freiem Gadolinium derart erfolgt, dass in der Zubereitung kein freies Gadolinium vorliegt,
d) die Komplexierung wird bei erhöhter Temperatur ausgeführt,
e) es wird weitere Base zugegeben, um den pH-Wert einzustellen und
f) das Endvolumen wird eingestellt.

2. Verfahren nach Anspruch 1, **dadurch gekennzeichnet, dass** zum Herstellen der Lösung gemäß Schritt a) zunächst DOTA in Wasser bei erhöhter Temperatur gelöst wird und dann Gadolinium zugegeben wird.

3. Verfahren nach Anspruch 1 oder 2, **dadurch gekennzeichnet, dass** das Komplexieren durch weiteres Zugeben einer Base wie L-Lysin oder Meglumin vervollständigt wird.

4. Verfahren nach einem der Anspruch 1 bis 3, **dadurch gekennzeichnet, dass** Schritt a) bei einer Temperatur zwischen 60°C und 95°C ausgeführt wird.

5. Verfahren nach einem der Ansprüche 1 bis 4, **dadurch gekennzeichnet, dass** die Lösung beim Ausführen der Schritte a) und/oder d) gerührt wird.

6. Verfahren nach einem der Ansprüche 1 bis 5, **dadurch gekennzeichnet, dass** in Schritt c) das Zugeben von freiem Gadolinium und/oder freiem DOTA in zwei oder mehr als zwei Teilmengen erfolgt.

7. Verfahren nach einem der Ansprüche 1 bis 6, **dadurch gekennzeichnet, dass** freies DOTA in Form einer Lösung und/oder freies Gadolinium in Form einer Lösung zugegeben wird.

8. Verfahren nach einem der Ansprüche 1 bis 7, **dadurch gekennzeichnet, dass** das Gadolinium als Oxid (Gd₂O₃) und in der Oxidationsstufe +III zugegeben wird.

9. Verfahren nach einem der Ansprüche 1 bis 8, **dadurch gekennzeichnet, dass** die Konzentration an freiem DOTA in der Zubereitung 180 - 2000 ppm, vorzugsweise 200 bis 1500 ppm, bezogen auf den Komplex, beträgt.

10. Verfahren nach einem der Ansprüche 1 bis 9, **dadurch gekennzeichnet, dass** die Konzentration an freiem Gadolinium in der Zubereitung unter 10 ppm liegt.

11. Verfahren nach einem der Ansprüche 1 bis 10, **dadurch gekennzeichnet, dass** der pH-Wert in Schritt e), insbesondere bei Raumtemperatur, auf einem Wert zwischen 7,0 und 7,2 eingestellt wird.

## Claims

1. Method for producing a liquid pharmaceutical preparation that contains a complex that consists of DOTA and gadolinium as well as a base, such as, for example, L-lysine or meglumine, **characterized by** the following steps:
a) An aqueous solution that consists of DOTA, gadolinium, and a base such as meglumine or L-lysine is produced,
b) In the solution obtained according to step a), the content of free DOTA and free gadolinium is determined,
c) Free gadolinium and/or free DOTA is/are added in order to adjust a content of free DOTA of 200 - 1500 ppm in the solution that is obtained according to step a), relative to the complex, whereby the addition of free DOTA and free gadolinium is carried out in such a way that there is no free gadolinium in the preparation,
d) The complexing is carried out at elevated temperature,
e) Further base is added in order to adjust the pH, and
f) The final volume is set.

2. Method according to Claim 1, **characterized in that** for producing the solution according to step a), first DOTA is dissolved in water at elevated temperature, and then gadolinium is added.

3. Method according to Claim 1 or 2, **characterized in that** the complexing is completed by further addition of a base such as L-lysine or meglumine.

4. Method according to one of Claims 1 to 3, **characterized in that** step a) is carried out at a temperature of between 60°C and 95°C.

5. Method according to one of Claims 1 to 4, **characterized in that** the solution is stirred when steps a) and/or d) is/are carried out.

6. Method according to one of Claims 1 to 5, **characterized in that** in step c), the addition of free gadolinium and/or free DOTA is carried out in two or more than two partial amounts.

7. Method according to one of Claims 1 to 6, **characterized in that** free DOTA is added in the form of a solution and/or free gadolinium is added in the form of a solution.

8. Method according to one of Claims 1 to 7, **characterized in that** the gadolinium is added as an oxide (Gd₂O₃) and in the oxidation stage +III.

9. Method according to one of Claims 1 to 8, **characterized in that** the concentration of free DOTA in the preparation is 180 - 2000 ppm, preferably 200 to 1500 ppm, relative to the complex.

10. Method according to one of Claims 1 to 9, **characterized in that** the concentration of free gadolinium in the preparation is less than 10 ppm.

11. Method according to one of Claims 1 to 10, **characterized in that** the pH in step e), in particular at room temperature, is adjusted to a value of between 7.0 and 7.2.

## Revendications

1. Méthode permettant de fabriquer une préparation pharmaceutique liquide, qui contient un complexe de DOTA et de gadolinium ainsi qu'une base, comme par exemple la L-lysine ou la méglumine, **caractérisée par** les étapes suivantes :
a) il est élaboré une solution aqueuse de DOTA, de gadolinium et d'une base comme la méglumine ou la L-lysine,
b) dans la solution obtenue selon l'étape a), la teneur en DOTA libre et en gadolinium libre est déterminée,
c) du gadolinium libre et/ou du DOTA libre est ajouté pour établir dans la solution obtenue selon l'étape a) une teneur en DOTA libre de 200 - 1500 ppm, en référence au complexe, l'addition de DOTA libre et de gadolinium libre ayant lieu de telle manière que dans la préparation il n'y aucune présence de gadolinium libre,
d) la mise en complexe est exécutée à température élevée,
e) autre base est ajoutée pour établir la valeur du pH et
f) le volume définitif est établi.

2. Méthode selon la revendication 1, **caractérisée en ce que** pour fabriquer la solution selon l'étape a), on dissout d'abord du DOTA dans de l'eau à température élevée et on ajoute ensuite du gadolinium.

3. Méthode selon la revendication 1 ou 2, **caractérisée en ce que** la mise en complexe est complétée par addition supplementaire d'une base comme la L-lysine ou la méglumine.

4. Méthode selon la revendication 1 à 3, **caractérisée en ce que** l'étape a) est exécutée à une température se situant entre 60°C et 95°C.

5. Méthode selon l'une quelconque des revendications 1 à 4, **caractérisée en ce que** la solution est agitée lors de l'exécution des étapes a) et/ou d).

6. Méthode selon l'une quelconque des revendications 1 à 5, **caractérisée en ce que** dans l'étape c), l'addition de gadolinium libre et/ou de DOTA libre a lieu en deux ou plus de deux quantités partielles.

7. Méthode selon l'une quelconque des revendications 1 à 6, **caractérisée en ce qu'**on ajoute du DOTA sous la forme d'une solution et/ou du gadolinium libre sous la forme d'une solution.

8. Méthode selon l'une quelconque des revendications 1 à 7, **caractérisée en ce que** le gadolinium est ajouté en tant qu'oxyde (Gd₂O₃) et dans la phase d'oxydation +III.

9. Méthode selon l'une quelconque des revendications 1 à 8, **caractérisée en ce que** la concentration en DOTA libre dans la préparation est de 180 - 2000 ppm, de préférence de 200 à 1500 ppm, en référence au complexe.

10. Méthode selon l'une quelconque des revendications 1 à 9, **caractérisée en ce que** la concentration en gadolinium libre dans la préparation est inférieure de 10 ppm.

11. Méthode selon l'une quelconque des revendications 1 à 10, **caractérisée en ce que** la valeur du Ph dans l'étape e), notamment à température ambiante, est établie à une valeur se situant entre 7,0 et 7,2.
